# EUROPEAN PATENT SPECIFICATION

(11) **EP 2 241 615 B1**
(45) Date of publication and mention of the grant of the patent: **12.11.2014**
(21) Application number: 09708024.6
(22) Date of filing: 03.02.2009
(51) Int. Cl.: C12N 1/20

(54) **MICROORGANISM-PROTECTING AGENT, AND METHOD FOR PRODUCTION OF FROZEN OR LYOPHILIZED MICROBIAL CELL**
MIKROORGANISMEN-SCHUTZMITTEL UND VERFAHREN ZUR HERSTELLUNG GEFRORENER ODER LYOPHILISIERTER MIKROBIELLER ZELLEN
AGENT PROTECTEUR DE MICROORGANISME ET PROCÉDÉ DE FABRICATION DE CELLULE MICROBIENNE CONGELÉE OU LYOPHILISÉE

(30) Priority: 07.02.2008 JP 2008027347
(43) Date of publication of application: 20.10.2010
(73) Proprietor: MEGMILK SNOW BRAND Co., Ltd., Higashi-ku Sapporo (JP)
(72) Inventor: AZUMA, Naoki, Kawagoe-shi Saitama 350-1165 (JP); WATANABE, Masayuki, Kawagoe-shi Saitama 350-1165 (JP); UENO, Hiroshi, Kawagoe-shi Saitama 350-1165 (JP); YOSHIOKA, Toshimitsu, Kawagoe-shi Saitama 350-1165 (JP)
(74) Representative: Andrae | Westendorp Patentanwälte Partnerschaft
(86) International application number: PCT/JP2009/051810
(87) International publication number: WO 2009/099075

(56) References cited:
- EP-A1- 0 442 522
- EP-A2- 0 576 780
- WO-A1-91/11509
- JP-A- 3 240 484
- JP-A- 5 336 952
- JP-T- 5 503 850
- MAITROT HENRI ET AL: "Production of concentrated freeze-dried cultures of Bifidobacterium longum in kappa-carrageenan-locust bean gum gel", BIOTECHNOLOGY TECHNIQUES, vol. 11, no. 7, 1997, pages 527-531, XP002663082, ISSN: 0951-208X
- BLANCHETTE,L. ET AL.: 'Protective effect of ultrafiltered retentate powder on stability of freeze-dried cultures of bifidobacteria during storage.' MILCHWISSENSCHAFT vol. 50, no. 7, 1995, pages 363 - 367, XP000519054
- GAGNE',J. ET AL.: 'Production of frozen and freeze-dried concentrates of Bifidobacterium infantis by membrane filtration.' MILCHWISSENSCHAFT vol. 48, no. 9, 1993, pages 501 - 505, XP000414546

## Description

### TECHNICAL FIELD

The present invention relates to a method of producing frozen or lyophilized microbial cells, a frozen or lyophilized mixture, and to a use of a permeate. The present, more specifically, relates to a method of producing frozen or lyophilized microbial cells characterized by mixing permeate obtained by filtering milk or whey using ultra-filtration (UF) membrane with microbial cells and freezing or lyophilizing it. In the present Description, the microbes belonging to Bifidobaterium is sometimes referred as bifidobacteria, and lactic acid bacteria other than bifidobacteria is sometimes referred as lactic acid bacteria, and the bifidobacteria and the lactic acid bacteria are sometimes mixed up to refer as lactic acid bacterial group.

### BACKGROUND ART

The bifidobacteria widely known as useful enterobacteria, is utilized widely in the food manufacturing, and is used in the production of milk products such as fermented milk and lactic acid bacterial beverages etc. Furthermore, in recent years, probiotics functions being bio-controlling functions have shown by ingestion of living microbial cells, such as inhibition effects on harmful enterobacteria and infection controlling effects (e.g., see Patent Document 1), immuno modulation effects (e.g., see Non Patent Document 1), and cholesterol-decreasing effects (e.g., see Non Patent Document 3), have been described one after another. Therefore, to maintain health by taking Bifidobacteria, many developments of fermented milk products and beverages as yogurt and cheese, a variety of foods and beverages containing lactic acid bacteria such as snacks and cakes, and many developments of utilization of them as raw materials for healthy foods and medicines, have been made.

When bifidobacteria is added to these foods and beverages, previously cultured bifidobacteria is processed and added. However, the process needs a large efforts and times, and there are many difficulties in techniques in order to maintain a high viability in the process.

On the other hands, lactic acid bacteria has been utilized as a starter for a variety of milk products such as yogurt and cheese and the like for a long time. And in recent years, these have been added to a variety of foods, in expectation of probiotics functions such as not only intestinal controlling effects but immuno modulation effects (e.g., see Non Patent Document 2) and improving of inflammatory intestinal disorder (e.g., see Non Patent Document 4).

However, although the microbial cells of these lactic acid bacteria are obtained by culturing milk and the like, there are problems of necessities of large efforts and long time and of taking enough care of quality control and the like of the microbial cells.

Therefore, it is possible to produce foods and beverages containing lactic acid bacteria such as milk products such as yogurt or cheese and the like easily, by using previously frozen or lyophilized living microbial cells of lactic acid bacteria. However, it is very difficult to manufacture the frozen or lyophilized lactic acid bacteria group having a microbial concentration needed for the products, because the damages or death of the microbial cells at the time of melting or lyophilizing have to be inhibited.

In the past, many freezing protective materials and lyophilizing protective materials were developed to dissolve the problems. As the materials, skim milk, monosodium glutamate, gelatin and sucrose (e.g., see Patent Document 1), phenylalanine, histidine, citric acid, succinic acid, tartaric acid and alkali carbonate (e.g., see Patent Document 2) and the like are known. As the other materials, lactose, trehalose, powdered skim milk, sorbitol, sodium L-ascorbate (e.g., see Non Patent Document 5) and the like are also known.
Patent Document 1: Japanese Patent publication No. 53-8792
Patent Document 2: Japanese Patent Laid-open No. 61-265085 Non Patent Document 1: A. Garcia-Lafuente et al.Modulation of colonic barrier function by the composition of the commensal flora in the rat.Got.48(4):503-507,2001.
Non Patent Document 2: E. Isolauri et al. Probiotics in the management of atopic eczema. Clinical & Experimental Allergy. 30(11):1604-1610, 2000.
Non Patent Document 3: J.Z.Xiao et al.Effects of milk Products fermented by Bifidobacterium longum on Blood Lipids in Rats and Healthy Adult Male Volunteers.Journal of Dairy Science.86:2452-2461, 2003.
Non Patent Document 4: K.L.Madsen et al.Lactobacillus species prevents colitis in interleukin 10-gene-deficient mice.Gastroenterology.116:1107-1114, 1999.
Non Patent Document 5: Ana S. Carvalho et al. Relevant factors for the preparation of freeze-dried lactic acid bacteria. International Dairy Journal.14(10):835-847,2004.

However, with these prior arts, damages or death of microbial cells at the time of freezing and lyophilizing were serious, and it was difficult to manufacture stable and highly concentrated frozen microbial cells or lyophilized microbial cells. Specifically, in a state where a microbial dispersion and a protective material in which microbial cells are assimilated at the time of microbial cells manufacturing are mixed, if the freezing process is not carried out in a possible low temperature and rapidly, the pH of the microbial cells dispersions which have to be frozen by acid produced by the active metabolism of microbial, will be lowered, and the viability will decrease remarkably. Further, the pH of the microbial cells dispersion at the time of freezing has remarkable influences on damages or death of the microbial cells at the time of melting and lyophilizing. So one has to take enough care of pH of the microbial cells dispersion before freezing, and has to neutralize it with alkali and the like depending on a case.

EP 0 576 780 A2 relates to a microorganism strain Lactobacillus casei ssp. rhamnosus LC-705, DSM 7061, having a yeast and mould controlling effect, to bacterial preparation comprising this strain, alone or in combination with a bacterium of the genus Propionibacterium or another strain of the bacterium Lactobacillus casei and/or with conventional agents used for yeast and mould control. In particular, Example 13 thereof describes the use of a preparation comprising Lactobacillus casei ssp. rhamnosus LC-705 strain for the inhibition of moulding of wheat bread.

H. Maitrot, C. Paquin, C. Lacroix and C. P. Champagne (Biotechnology Techniques, Vol. 11, No. 7, July 1997, pp. 527-531) describe the production of concentrated freeze-dried cultures of Bifidobacterium longum in κ-carrageenan-locust bean gum gel.

EP 0 442 522 A1 discloses a method for culturing a chlorella aerobically in a culture medium containing an organic carbon source, which comprises using whey treated with a lactose-decomposing enzyme as the said organic carbon source.

L. Blanchette, D. Roy, and S. F. Gauthier (Milchwissenschaft 50(7) 1995, p. 363-367) discuss in a publication the effect of ultrafiltered retentate powder on stability of freeze-dried cultures of bifidobacteria during storage.

J. Gagné, D. Roy and S. F. Gauthier (Milchwissenschaft 48(9) 1993, p. 501-505) describe the production of frozen and freeze-dried concentrates of Bifidobacterium infantis by membrane filtration.

Further, in the process of manufacturing frozen or lyophilized microbial cells, the speed of freezing microbial cell dispersion has remarkable influences on the damages or death of the microbial cells. That is, it is desirable to decrease the liquid temperature as rapid as possible, but when manufacturing on an industrial scale, there are many problems to be dissolved on carrying out the rapid freezing.

### DISCLOSURE OF THE INVENTION

### PROBLEM TO BE SOLVED BY THE INVENTION

As described above, there is no general-purpose protective agent being able to inhibit the damages or death of microbial cells in a freezing and lyophilizing of microbe process, and the developments of such agents have been expected. Therefore, in view of the problems, the purpose of the present invention is to provide a new viability-improving agent which has an excellent effect on the improvement of viability of the microbe and has no bad effect on the flavor of the products and which can be produced at low manufacturing cost.

### MEANS OF SOLVING THE PROBLEM

On the other hand, when cheese is prepared, a large amount of cheese whey is excreted as a by-product. The whey has peculiar flavor, and it does not suit to a beverage as it is. So whey is mainly used for the preparation of WPC in which protein is concentrated from the whey and of Whey Protein Isolate (WPI) in which protein is concentrated and dried after carrying out ion-exchange resin adsorption method. These are excellent in amino acid composition compared with casein, and have high nutritive values, and further, these are used widely as cheap food materials. However, in recent years, with the increase of cheese consumption, the amount of waste liquid which are produced at WPC and WPI manufacturing, are increased, so a development of an utilizing method is further needed in the lights of the environmental protection and these effective utilizations.

The present inventors focus on that the waste liquid produced at the time of WPC and WPI manufacturing can be available in a high amount and at a low cost. On the other hand, in order to dissolve the above problems, as a result of the earnest research on a method of preparing the above-mentioned lyophilized microbial cells, the inventors found that the damages or death of the microbial cells can be inhibited in the freezing and lyophilizing process, by mixing microbial cells and a permeate which is produced by filtering milk or whey by ultra-filtration (UF) membrane with microbial cells and freezing or freeze-drying it, and thus completed the present invention.

Disclosed herein is:
(1) A method of preparing frozen or lyophilized microbial cells **characterized in that** permeate obtained by filtering milk or whey with ultra-filtration (UF) membrane is mixed with microbial cells, and the mixture is frozen or lyophilized.
(2) A method of preparing frozen or lyophilized microbial cells **characterized in that** lactose in permeate obtained by filtering milk or whey with ultra-filtration (UF) membrane is decomposed with enzyme and/or heat, then the permeate is mixed with microbial cells, and the mixture is frozen or lyophilized.
(3) The method of preparing **characterized in that** the microbes are one or more of Bifidobacterium, Lactobacillus, Streptococcus, and Lactococcus.
(4) The frozen or lyophilized microbial cells which can be prepared by the above method.
(5) Frozen or lyophilized microbial cells which can be prepared by mixing permeate obtained by filtering milk or whey by ultra-filtration (UF) membrane with one or more of microbial cells of Bifidobacterium, Lactobacillus, Streptococcus and Lactococcus, and freezing or lyophilizing the mixture.
(6) Frozen or lyophilized microbial cells prepared by decomposing lactose in a permeate which is obtained by filtering milk or whey by ultra-filtration (UF) membrane with enzyme and/or heat, and then mixing the permeate with one or more of microbial cells of Bifidobacterium, Lactobacillus, Streptococcus and Lactococcus and freezing and lyophilizing the mixture.
(7) A protective agent for frozen or lyophilized microbial cells which contains permeate obtained by filtering milk or whey by ultra-filtration (UF) membrane as an active component.

The present invention relates to the invention having the following constitutions.

According to one aspect, there is provided a method of preparing frozen or lyophilized microbial cells, as defined in claim 1 annexed.

According to another aspect, there is provided a method of preparing frozen or lyophilized microbial cells, as defined in claim 2 annexed.

According to another aspect, there is provided a frozen or lyophilized mixture, as defined in claim 5 annexed.

According to another aspect, there is provided a frozen or lyophilized mixture, as defined in claim 6 annexed.

According to another aspect, there is provided a use of a permeate obtained by filtering milk or whey by ultrafiltration (UF) membrane as an active component of a protective agent for frozen or lyophilized microbial cells, as defined in claim 7 annexed.

According to another aspect, there is provided a use of a permeate produced by filtering milk or whey by ultrafiltration (UF) membrane for inhibiting damages or death of microbial cells in the freezing and/or lyophilizing process.

### EFFECTS OF THE INVENTION

The present invention provides a method of producing frozen or lyophilized microbial cells which can inhibit damages or death of cells and have high viabilities in freezing or lyophilizing process.

### BEST MODE FOR CARRYING OUT THE INVENTION

The characteristic of the method of production of the present invention, is to mix a permeate obtained by filtering milk or whey using ultra-filtration (UF) membrane with microbial cells, and to freeze or lyophilize it. The invention is explained in detail below.

As the raw material used for the production of permeate in the present invention, milk or whey may be exemplified. As milk, any milk which is usually used in food manufacturing may be used. For example, whole milk, non and reduced fat milk, reconstituted milk, condensed milk, butter milk, cream, powdered skim milk, or mixtures thereof can be exemplified.

As whey, by-products obtained when cheese is prepared from cow's milk and milk of mammals such as water buffalo and goat, by-products obtained when acid casein is prepared by acidifying the pH of milk, and permeates obtained by treating milk with fine filtration membrane, can be used. If these are acidic, these can be used after re-controlling the pH to neutral with a pH controlling agent such as sodium hydroxide, sodium carbonate, potassium hydroxide. These can be used in powder, but when these are reacted with enzyme, these can be used in a form of water solution.

By filtering these raw materials using ultra-filtration (UF) membrane, permeates can be obtained. For the membrane treatments, an ultra-filtration (UF) membrane having 5,000 or 10,000 molecular weight cutoff, can be used. The permeates thus obtained contain mainly 8 to 20g/100g lactose, and low molecular weight materials such as minerals, vitamins, amino acids, peptides, and the like.

As minerals, water-soluble minerals contained in milk, for example, 20mg to 1,000mg/100g of calcium, 30 to 1,000mg/100g of potassium, 30 to 300mg/100g or phosphorus, 10 to 100mg/100g of magnesium and the like, may be exemplified.

As vitamins, vitamins contained in milk, for examples, 0. 04mg to 2mg/100g of vitamin B₂, 0.01mg to 0.5mg/100g of vitamin B₁, 0.03mg to 1.5mg/100g of niacin, 0.5mg to 25mg/100g of vitamin C and the like may be exemplified.

The obtained permeate can be used in a form of water solution as it is, or can be powdered by concentrating or drying and then mixed with microbial cells so as to prepare frozen or lyophilized microbial cells.

As a modification of the frozen or lyophilized protective agent, the obtained permeate can be used in a water solution as it is or can be powdered by concentrating or drying and mixed with microbial cells so as to prepare frozen or lyophilized microbial cells. When it is powdered, it has advantages in the light of preservation and transportation and the like. Further, after the powdered materials are formed into a solution again, it can be mixed with microbial cells.

The microbes adapted for the microbial protective agent, are useful microbes which are used after freezing or lyophilizing, and the kinds thereof are not limited specifically. However, for examples, bifidobacteria, lactic acid bacteria, microbes, molds, yeast and the like may be exemplified. More specifically, as bifidobacteria, a microbe belonging to Bifidobacterium, for examples, Bifidobacterium longum and the like can be exemplified. However, the bifidobacteria used is not limited to these microbe species.

The lactic acid bacteria group is microbes generally classified as lactic acid bacteria, microbes belonging to Lactobacillus, specically Lactobacillus gasseri, Lactobacillus delbrueckii ssp. bulgaricus and the like, microbes belonging to Streptococcus, specially, Streptococcus thermophilus, and microbes belonging to Lactococcs, specially, Lactococcus lactis ssp. lactis, may be exemplified. However, the lactic acid bacteria group used is not limited to these bacteria.

As microbes other than lactic acid bacteria group, microbes belonging to Propionibacterium, specifically, Propionibacterium freudenreichii and the like, and microbes belonging to Bacillus, specifically Bacillus subtilis and the like, may be exemplified. As molds, microbes belonging to Penicillium, specifically Penicillium roqueforti and the like, may be exemplified. As yeasts, microbes belonging to Saccharomyces, specifically Saccharomyces cerevisiae and the like, may be exemplified. However, the bacteria, molds, and yeasts which can be used, are not limited to these species.

The microbial cells of the microbes can be prepared by conventional methods. For examples, one or 2 or more of the afore-mentioned microbes are cultured in a liquid medium containing glucose, yeast extract, peptone and the like at a temperature usually from 25 to 45°C for 4 to 24 hours, and the cultured microbial cells are collected from the liquid medium to obtain wet microbial cells after treatments such as washing and the like.

To the obtained wet microbial cells, the permeate is usually added in a concentration of from 10 to 75%, preferably from 25 to 50%, and mixed uniformly.

After mixing, freezing or lyophilizing can be carried out by a conventional manner. When freezing, for example, freezing can be carried out -20°C to -160°C (using liquid nitrogen and the like), and when lyophilizing, it can be carried out at a tray temperature at 35°C or less under a vacuum of about 1.0X10⁻¹torr.

Otherwise, after decomposing the lactose in the permeate which is obtained by filtering milk or whey with ultra-filtration (UF) membrane, the permeate can be mixed with microbe. The lactose decomposition is preferably carried out using β -galactosidase.

In the present invention, the permeate treated by membrane is added and mixed in order to obtain frozen or lyophilize microbial cells which can inhibit damages or death of microbial cells and have high viabilities in freezing or lyophilizing process.

### EXAMPLE

The Examples of the present invention are shown below to explain the present invention in detail.

The Examples below-descried are intended to only explain the present invention, and are not intended to limit the scope of the present invention to the description of the Examples.

### Example 1

### (Preparation of lactic acid bacteria group)

In GAM medium (manufactured by Nihon Seiyaku KK) in which glucose was added to have a final concentration of 1%, 2% of pre-culture of Bifidobacterium longum JCM 1217^{T} was inoculated, and cultured at 37°C for 16 hours to obtain 1000ml of bifidobacteria suspension. After cooling 250ml of the microbial suspension, it was poured into a 500ml centrifuge tube to centrifuge at 5000rpm for 10 minutes, and supernatant was removed in an appropriate amount by decantation to obtain bifidobacteria suspension in which the bifidobacteria was concentrated to 25 times.

### (Preparation of permeate)

After centrifuging 80kg of cheese whey using cream separator, it was sterilized at 65°C for 30 minutes, and concentrated to 19 times using UF membrane having 10,000 of molecular weight fractionated (PW1812T, material: poly ether sulphone, module: spiral type, membrane area: 0.55m², manufactured by DESALINATION) at a circulation flow rate of 10L/min., under an average pressure of 4kg/cm² to obtain 75kg of a UF permeate having 14% of solid, 11% of lactose, 0.3% of protein and 0.6% of minerals. The obtained permeate can be used as it is, as a frozen or lyophilized protective agent of the present invention.

### (Preparation of lyophilized microbial cells)

In the prepared bifidobacteria suspension, water (Sample 1), a mixture liquid containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), and permeate prepared in Example 1 (Sample 4), were added in the same amount to have four each bifidobacteria suspension in which bifidobacteria was concentrated to 12.5 times.

The obtained each microbial suspension was lyophilized by a conventional method (Kyowa vacuum lyophilized apparatus RLE-308, manufactured by Kyowa Shinku Gijutsu KK, tray temperature: 35°C or less, degree of vacuum: 1.0x10⁻¹torr) to obtain about 3g of each lyophilized bifidobacteria microbial cells.

Using the obtained lyophilized bifidobacteria microbial cells, a forced deterioration test was carried out at 37°C, and after three weeks from the beginning of the test, the number of the survival bacteria was counted in each Sample, and the viabilities were calculated. The results are shown in Table 1.

### Example 2

### (Preparation of lactic acid bacteria group)

In M17 medium (manufactured by DIFCO) in which lactose was added to have a final concentration of 0.5%, 2% of pre-culture of Lactococcus lactis ssp.lactis JCM5808^{T} was inoculated, and cultured at 30°C for 16 hours so as to obtain 1000ml of lactic acid bacteria suspension. The microbial suspension was treated in the same manner as described in Example 1, lactic acid bacteria suspension in which the lactic acid bacteria was concentrated to 25 times, was obtained.

### (Preparation of permeation)

In the bacteria suspension, water (sample 1), a mixture solution containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), and the permeate prepared in Example 1 (Sample 4), were added in each equal amount to obtain four types of each bifidobacteria suspension in which lactic acid bacteria was concentrated to 12.5 times.

The obtained each microbial suspension was lyophilized by a conventional method to obtain about 3g of each lyophilized lactic acid bacteria microbial cells.

Using the obtained lyophilized lactic acid bacteria microbial cells, a forced deterioration test was carried out at 37°C, and after three weeks from the beginning of the test, the number of the survival bacteria was counted in each Sample, and the viabilities were calculated. The results are shown in Table 1.

### Example 3

### (Preparation of lactic acid bacteria gorup)

In MRS medium (manufactured by DIFCO), 2% of pre-culture of Lactobacillus delbrueckii ssp.bulgaricus JCM 1002^{T}, or Lactobacillus gasseri JCM 1131^{T}, or Lactobacillus casei JCM 1134^{T} or Lactobacillus plantarum JCM 1149^{T} was inoculated, and cultured at 37°C for 16 hours so as to obtain 1, 000ml of lactic acid bacteria suspension. With the same treatment of the bacteria suspension as described in Example 1, each lactic acid bacteria suspension in which lactic acid bacteria was concentrated to 25 times, was obtained.

### (Preparation of lyophilized microbial cells)

In the bacteria liquid, water (Sample 1), a mixture solution containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), and the permeate prepared in Example 1(Sample 4), were added in equal amount to obtain four types of each lactic acid bacteria suspension in which lactic acid bacteria was concentrated to 12.5 times.

The obtained each microbial suspension was lyophilized by a conventional method to obtain about 3g of each lyophilized lactic acid bacteria microbial cells.

Using each lyophilized lactic acid bacteria microbial cells, a forced deterioration test was carried out at 37°C, and after three weeks from the beginning of the test, the number of the survival bacteria was counted in the each Sample, and the viabilities were calculated. The results are shown in Table 1.

**Table 1**

| Viability of each sample under forced deterioration test at 37°C for three weeks (unit:%) | | | | |
|---|---|---|---|---|
| | Sample 1 | Sample 2 | Sample 3 | Sample 4 |
| B. longum JCM 1217^{T} | 0.08 | 0,30 | 0.86 | 1.22 |
| Lc. lactis JCM 5805^{T} | 30.30 | 55.25 | 70.12 | 85.65 |
| Lb. bulgaricus JCM 1002^{T} | < 0.01 | 0.16 | 0.83 | 1.12 |
| Lb. gasseri JCM 1131^{T} | < 0.01 | < 0.01 | 0.12 | 0.32 |
| Lb. casei JCM 1134^{T} | < 0.01 | < 0.01 | 0.05 | 0.10 |
| Lb. plantarum JCM 1149^{T} | 0.17 | 0.51 | 0.78 | 0.99 |

From the results shown in Table 1, it was confirmed for every lactic acid bacteria group that Sample 4 in which the membrane-treated permeate was used had a high viability compared with Sample 1 in which the protective agent was not used, and with Sample 2 and 3 in which the known protective agents were used. Sample 4 of the present invention contained almost the same amount of lactose as Sample 3, but showed high viability compared with Sample 3. It was confirmed that the membrane-treated permeate had a superiority on the effects on the viability.

### Example 4

### (Preparation of lactic acid bacteria group)

With the same method as described in Example 1, microbial suspension of Bifidobacterium longum JCM 1217^{T} was obtained in which it was concentrated to 25 times.

### (Preparation of permeate)

With the same method as described in Example 1, 75kg of UF permeate was obtained. Then, the obtained permeate was concentrated by concentration under a reduced pressure so as to have 65 weight% of whole solid content. 25g of β-galactosidase (Sumilacto L, manufactured by Shinnihon kagaku kogyo KK) was added to the obtained 5kg of the concentrated UF membrane-treated permeate, an enzyme reaction was carried out at 55°C for two hours, then it was heated to 85°C for five minutes using a plate type heat exchanger having hold tube to stop the enzyme reaction. Thus, 4kg of lactose-decomposed syrup composition was obtained. The obtained permeate can be used as the freezing or lyophilizing protective agent of the invention as it is.

### (Preparation of lyophilized microbial cells)

In each of the prepared Bifidobacteria microbial suspension, water (Sample 1), a mixture liquid containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), and lactose- decomposed syrup composition (Sample 5), were added in equal amount to obtain four type of each bifidobacteria suspension in which lactobacillus bifidas was concentrated to 12.5 times.

The obtained each microbial suspension was lyophilized by the same method as described in Example 1 so as to obtain about 3g of each lyophilized bifidobacteria microbial cells.

Using the lyophilized bifidobacteria microbial cells, a forced deterioration test was carried out at 37°C, and after three weeks from the beginning of the test, the number of the survival bacteria was counted in each Sample, and the viabilities were calculated. The results are shown in Table 2.

### Example 5

### (Preparation of lactic acid bacteria group)

With the same method as described in Example 2, microbial suspension of Lactococcus lactis ssp. lactis JCM 5805^{T} was obtained in which it was concentrated to 25 times.

### (Preparation of lyophilized microbial cells)

In the microbial suspension, water (Sample 1), a mixture solution containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), or lactose-decomposed syrup composition prepared in Example 4 (Sample 5), were added in equal amount to obtain four types of each lactic acid bacteria microbial suspension in which lactic acid bacteria was concentrated to 12.5 times.

The obtained each microbial suspension was lyophilized by the same method as described in Example 1 to obtain about 3g of each lyophilized lactic acid bacteria microbial cells.

Using the lyophilized lactic acid bacteria microbial cells, a forced deterioration test was carried out at 37°C. After three weeks from the beginning of the test, the number of the survival bacteria was counted in each Sample, and the viabilities were calculated. The results are shown in Table 2.

### Example 6

### (Preparation of lactic acid bacteria group)

With the same method as described in Example 3, each microbial suspension of Lactobacillus delbrueckii ssp. Bulgaricus JCM 1002^{T}, or Lactobacillus gasseri JCM 1131^{T}, or Lactobacillus plantarum JCM 1149^{T} was obtained in which microbial suspension was concentrated to 25 times.

### (Preparation of lyophilized microbial cells)

In the microbial suspension, water (Sample 1), a mixture solution containing 10% of powdered skim milk (manufactured by Snow Brand Milk Products KK) and 1% of L-monosodium glutamate (manufactured by Wako Junyaku KK) (Sample 2), 10% water solution of lactose (manufactured by Wako Junyaku KK) (Sample 3), or lactose-decomposed syrup composition prepared in Example 4 (Sample 5), were added in equal amount to obtain four types of each lactic acid bacteria microbial suspension in which lactic acid bacteria was concentrated to 12.5 times.

The obtained each microbial suspension was lyophilized by the same method as described in Example 1 to obtain about 3g of each lyophilized lactic acid bacteria microbial cells.

Using the lyophilized lactic acid bacteria microbial cells, a forced deterioration test was carried out at 37°C. After three weeks from the beginning of the test, the number of the survival bacteria is counted in each Sample, and the viabilities were calculated. The results are shown in Table 2.

**Table 2**

| Viability of each Sample under forced deterioration test at 37°C for three weeks (unit:%) | | | | |
|---|---|---|---|---|
| | Sample 1 | Sample 2 | Sample 3 | Sample 5 |
| B. longum JCM 1217^{T} | 0.08 | 0.30 | 0.86 | 0.95 |
| Lc. lactis JCM 5805^{T} | 30.30 | 55.25 | 70.12 | 82.66 |
| Lb. bulgaricus JCM 1002^{T} | < 0.01 | 0.16 | 0.83 | 1.55 |
| Lb. gasseri JCM 1131^{T} | < 0.01 | < 0.01 | 0.12 | 0.25 |
| Lb. plantarum JCM 1149^{T} | 0.17 | 0.51 | 0.78 | 0.95 |

From the results shown in Table 2, it was confirmed that Sample 5 in which the membrane-treated permeate was used, had high viabilities for all lactic acid bacteria, compared with Sample 1 in which protective agent was not used and with Samples 2 and 3 in which known protective agents were used.

## Claims

1. A method of preparing frozen or lyophilized microbial cells
**characterized in that** said method comprises
obtaining a permeate by filtering milk or whey using ultra-filtration (UF) membrane,
the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C,
and **in that** the permeate obtained by filtering milk or whey with ultra-filtration (UF) membrane is mixed with microbial cells and the mixture is frozen or lyophilized.

2. A method of preparing frozen or lyophilized microbial cells
**characterized in that** said method comprises
obtaining a permeate by filtering milk or whey using ultra-filtration (UF) membrane,
the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C,
and **in that** lactose in the permeate obtained by filtering milk or whey with ultra-filtration (UF) membrane is decomposed with enzyme and optionally heat, and then the permeate is mixed with microbial cells and the mixture is frozen or lyophilized.

3. The method of preparing of Claim 1 or 2 **characterized in that** the microbes are one or more of Bifidobacterium Lactobacillus, Streptococcus, and Lactococcus.

4. The method of preparing frozen or lyophilized microbial cells according to any of claims 1 to 3, wherein the obtained permeate is used in a form of water solution as it is or is powdered by concentrating or drying.

5. Frozen or lyophilized mixture which can be prepared by
obtaining a permeate by filtering milk or whey using ultra-filtration (UF) membrane, the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C,
mixing the permeate obtained by filtering milk or whey by ultra-filtration (UF) membrane with one or more of microbial cells of Bifidobacterium, Lactobacillus, Streptococcus and Lactococcus, and
freezing or lyophilizing the mixture.

6. Frozen or lyophilized mixture prepared by
obtaining a permeate by filtering milk or whey using ultra-filtration (UF) membrane,
the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C,
decomposing lactose in the permeate which is obtained by filtering milk or whey by ultra-filtration (UF) membrane with enzyme and/or heat, and
then mixing the permeate with one or more of microbial cells of Bifidobacterium, Lactobacillus, Streptococcus and Lactococcus and freezing or lyophilizing the mixture.

7. Use of a permeate obtained by filtering milk or whey by ultrafiltration (UF) membrane as an active component of a protective agent for frozen or lyophilized microbial cells,
said use comprising mixing the permeate with microbial cells, and freezing or lyophilizing the mixture,
the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C.

8. Use of a permeate produced by filtering milk or whey by ultrafiltration (UF) membrane for inhibiting damages or death of microbial cells in the freezing and/or lyophilizing process,
the permeate obtained by filtering milk or whey with ultra-filtration (UF) membrane containing 8 to 20g/100g lactose, minerals comprising 20mg to 1,000mg/100g of calcium, 30 to 1,000mg/100g of potassium, 30 to 300mg/100g of phosphorus and 10 to 100mg/100g of magnesium, and vitamins comprising 0.04mg to 2mg/100g of vitamin B₂, 0.01mg to 0.5mg/100g of vitamin B₁, 0.03mg to 1.5mg/100g of niacin, 0.5mg to 25mg/100g of vitamin C.

## Patentansprüche

1. Verfahren zur Herstellung von gefrorenen oder lyophilisierten mikrobiellen Zellen, **dadurch gekennzeichnet, dass** das Verfahren umfasst
Erhalten eines Permeats durch Filtrieren von Milch oder Molke unter Verwendung einer Ultrafiltration (UF)-Membran, wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält,
und dass das Permeat, das durch Filtrieren von Milch oder Molke mit einer Ultrafiltration (UF)-Membran erhalten wird, mit mikrobiellen Zellen gemischt wird und das Gemisch gefroren oder lyophilisiert wird.

2. Verfahren zur Herstellung von gefrorenen oder lyophilisierten mikrobiellen Zellen, **dadurch gekennzeichnet, dass** das Verfahren umfasst
Erhalten eines Permeats durch Filtrieren von Milch oder Molke unter Verwendung einer Ultrafiltration (UF)-Membran, wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält,
und dass Lactose in dem Permeat, das durch Filtrieren von Milch oder Molke mit einer Ultrafiltration (UF)-Membran erhalten wird, mit einem Enzym und gegebenenfalls Wärme zersetzt wird und dann das Permeat mit mikrobiellen Zellen gemischt wird und das Gemisch gefroren oder lyophilisiert wird.

3. Verfahren zur Herstellung nach Anspruch 1 oder 2, **dadurch gekennzeichnet, dass** die Mikroben eine oder mehrere von Bifidobacterium, Lactobacillus, Streptococcus und Lactococcus sind.

4. Verfahren zur Herstellung von gefrorenen oder lyophilisierten mikrobiellen Zellen gemäß einem der Ansprüche 1 bis 3, wobei das erhaltene Permeat in einer Form einer Wasserlösung so wie sie vorliegt verwendet wird oder durch Konzentrieren oder Trocknen in Pulverform überführt wird.

5. Gefrorenes oder lyophilisiertes Gemisch, welches hergestellt werden kann durch
Erhalten eines Permeats durch Filtrieren von Milch oder Molke unter Verwendung einer Ultrafiltration (UF)-Membran, wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält,
Mischen des Permeats, das durch Filtrieren von Milch oder Molke durch eine Ultrafiltration (UF)-Membran erhalten wird, mit einer oder mehreren mikrobiellen Zellen von Bifidobacterium, Lactobacillus, Streptococcus und Lactococcus, und Gefrieren oder Lyophilisieren des Gemisches.

6. Gefrorenes oder lyophilisiertes Gemisch, hergestellt durch
Erhalten eines Permeats durch Filtrieren von Milch oder Molke unter Verwendung einer Ultrafiltration (UF)-Membran, wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält,
Zersetzen von Lactose in dem Permeat, das durch Filtrieren von Milch oder Molke durch eine Ultrafiltration (UF)-Membran erhalten wird, mit einem Enzym und/oder Wärme, und
dann Mischen des Permeats mit einer oder mehreren mikrobiellen Zellen von Bifidobacterium, Lactobacillus, Streptococcus und Lactococcus und Gefrieren oder Lyophilisieren des Gemisches.

7. Verwendung eines Permeats, das durch Filtrieren von Milch oder Molke durch eine Ultrafiltration (UF)-Membran erhalten wird, als eine aktive Komponente eines Schutzmittels für gefrorene oder lyophilisierte mikrobielle Zellen, wobei die Verwendung Mischen des Permeats mit mikrobiellen Zellen und Gefrieren oder Lyophilisieren des Gemisches umfasst,
wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält.

8. Verwendung eines Permeats, das durch Filtrieren von Milch oder Molke durch eine Ultrafiltration (UF)-Membran hergestellt wird, zum Einschränken von Schäden oder Tod von mikrobiellen Zellen in dem Gefrier- und/oder Lyophilisierverfahren,
wobei das Permeat, das durch Filtrieren von Milch oder Molke mit einer Ultrafiltration (UF)-Membran erhalten wird, 8 bis 20 g/100 g Lactose, Mineralien, umfassend 20 mg bis 1.000 mg/100 g Calcium, 30 bis 1.000 mg/100 g Kalium, 30 bis 300 mg/100 g Phosphor und 10 bis 100 mg/100 g Magnesium, und Vitamine, umfassend 0,04 mg bis 2 mg/100 g Vitamin B₂, 0,01 mg bis 0,5 mg/100 g Vitamin B₁, 0,03 mg bis 1,5 mg/100 g Niacin, 0,5 mg bis 25 mg/100 g Vitamin C, enthält.

## Revendications

1. Procédé de préparation de cellules microbiennes congelées ou lyophilisées, **caractérisé en ce que** le procédé comprend l'obtention d'un perméat par filtration de lait ou de lactosérum à l'aide d'une membrane d'ultra-filtration (UF), le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C,
et **en ce que** le perméat obtenu par filtration de lait ou de lactosérum à l'aide d'une membrane d'ultra-filtration (UF) est mélangé à des cellules microbiennes et que le mélange est congelé ou lyophilisé.

2. Procédé de préparation de cellules microbiennes congelées ou lyophilisées, **caractérisé en ce que** le procédé comprend l'obtention d'un perméat par filtration de lait ou de lactosérum à l'aide d'une membrane d'ultra-filtration (UF), le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C,
et **en ce que** le lactose dans le perméat obtenu par filtration de lait ou de lactosérum à l'aide d'une membrane d'ultra-filtration (UF) est décomposé par une enzyme et le cas échéant, la chaleur, puis le perméat est mélangé à des cellules microbiennes et que le mélange est congelé ou lyophilisé.

3. Procédé de préparation selon la revendication 1 ou 2, **caractérisé en ce que** les microbes sont un ou plusieurs parmi les *Bifidobacterium, Lactobacillus, Streptococcus* et *Lactococcus.*

4. Procédé de préparation de cellules microbiennes congelées ou lyophilisées selon l'une quelconque des revendications 1 à 3, où le perméat obtenu est utilisé sous forme d'une solution aqueuse telle quelle ou il est pulvérisé par concentration on séchage.

5. Mélange congelé ou lyophilisé, qui peut être préparé par :
obtention d'un perméat par filtration de lait ou de lactosérum à l'aide d'une membrane d'ultra-filtration (UF), le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C,
mélange du perméat obtenu par filtration de lait ou de lactosérum à l'aide d'une membrane d'ultra-filtration (UF) à une ou plusieurs cellules microbiennes parmi les *Bifidobacterium, Lactobacillus, Streptococcus* et *Lactococcus,* et
congélation ou lyophilisation du mélange.

6. Mélange congelé ou lyophilisé, qui peut être préparé par :
obtention d'un perméat par filtration de lait ou de lactosérum à l'aide d'une membrane d'ultra-filtration (UF), le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C,
décomposition du lactose dans le perméat qui est obtenu par filtration de lait ou de lactosérum à l'aide d'une membrane d'ultra-filtration (UF), avec une enzyme et/ou la chaleur, et
mélange du perméat à une ou plusieurs cellules microbiennes parmi les *Bifidobacterium, Lactobacillus, Streptococcus* et *Lactococcus,* et
congélation ou lyophilisation du mélange.

7. Utilisation d'un perméat obtenu par filtration de lait ou de lactosérum à l'aide d'une membrane d'ultra-filtration (UF) en tant que composant actif d'un agent protecteur pour cellules microbiennes congelées ou lyophilisées, ladite utilisation comprenant le mélange du perméat avec des cellules microbiennes, et la congélation ou la lyophilisation du mélange, le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C.

8. Utilisation d'un perméat produit par filtration de lait ou de lactosérum à l'aide d'une membrane d'ultra-filtration (UF) pour inhiber les dommages ou la mort de cellules microbiennes dans le processus de congélation et/ou de lyophilisation,
le perméat obtenu par filtration de lait ou de lactosérum à l'aide d'une membrane d'ultra-filtration (UF) contenant 8 à 20 g/100 g de lactose, des minéraux comprenant 20 mg à 1000 mg/100 g de calcium, 30 mg à 1000 mg/100 g de potassium, 30 mg à 300 mg/100 g de phosphore et 10 mg à 100 mg/100 g de magnésium, et des vitamines comprenant 0,04 mg à 2 mg/100 g de vitamine B2, 0,01 mg à 0,5 mg/100 g de vitamine B1, 0,03 mg à 1,5 mg/100 g de niacine, 0,5 mg à 25 mg/100 g de vitamine C.
